# EUROPEAN PATENT APPLICATION

(11) **EP 4 389 048 A1**
(43) Date of publication of application: **26.06.2024**
(21) Application number: 23218419.2
(22) Date of filing: 20.12.2023
(51) Int. Cl.: A61B 34/20, A61B 34/10, A61B 90/00

(54) **TOOL NAVIGATION IN MIXED REALITY COMPUTER-ASSISTED SURGERY**

(30) Priority: 20.12.2022 US 202263476203 P
(71) Applicant: Orthosoft ULC, Montreal, QC H3C 2N6 (CA)
(72) Inventor: COUTURE, Pierre, Montreal, H4C 2J8 (CA); CASAUBON, Jerome, Montreal, H1Y 3A1 (CA); AMIOT, Louis-Philippe, Montreal, H3C 2N6 (CA); MERETTE, Jean-Sébastien, Mont-St-Hilaire, J3H 4W2 (CA)
(74) Representative: Taor, Simon Edward William

(57) **Abstract**

A system for tracking a surgical tool relative to a bone in computer-assisted surgery, comprising: a processing unit; and a non-transitory computer-readable memory communicatively coupled to the processing unit and comprising computer-readable program instructions executable by the processing unit for: tracking the surgical tool relative to the bone; merging virtual models of the surgical tool and the bone to the surgical tool and the bone in the tracking; calculating a location of a working end of the surgical tool relative to the bone using the tracking, in a concealed condition of the working end of the surgical tool relative to the bone; and outputting the location of the working end of the surgical tool relative to the bone.

## Description

### CROSS-REFERENCE TO RELATED APPLICATION

The present application claims the priority of United States Patent Application No. 63/476,203, filed on December 20, 2022 and incorporated herein in its entirety.

### TECHNICAL FIELD

The present application relates to bone and tool tracking in computer-assisted orthopedic surgery and in robotized computer-assisted surgery.

### BACKGROUND OF THE ART

The navigation of surgical instruments or tools is an integral part of computer-assisted surgery (hereinafter "CAS"). The tools are navigated, i.e., tracked for position and/or orientation, in such a way that relative information pertaining to bodily parts is obtained. The information may be used in various interventions (e.g., orthopedic surgery, neurological surgery) with respect to the body, such as bone alterations, implant positioning, incisions and the like during surgery.

In orthopedic surgery, optical tracking technology is commonly used. Depending on the optical tracking technology used, different types of trackable members are fixed, permanently or temporarily, to the item that needs to be tracked. For instance, trackable members are fixed to the limbs and to the different surgical instruments, and these trackable members are tracked by the tracking system. The CAS system calculates position and orientation data associated with the tracking, and the information displayed by the computer is used by the surgeon to visualize the position of the instrument(s) being manipulated with respect to the limbs, or in numerical values.

In optical tracking technology, the optical elements must be in the line of sight of the optical sensor device. One common constraint with optical tracking systems is the requirement for a line of sight between stationary image acquisition devices and the objects to track. Accordingly, surgery employing optical tracking may be imposed a given orientation as a function of the required visibility between the optical sensor apparatus and the optical elements. If the line of sight is disrupted, optical tracking may be paused, as a possible consequence. In automated robotic surgery, the interruption of optical tracking may result in the need for human intervention. There remains room for improvement.

Moreover, an ongoing challenge in orthopedic surgery is the lack of visibility of bone alterations/resections as surgical procedures opt increasingly more for minimally invasive approaches. To reduce invasiveness, surgical procedures involve smaller incisions, to limit soft tissue wounds. As a result, bone and tool visibility may be reduced. However, there are some limits to minimally invasive surgery, as the surgeon may require a greater bone exposure to properly perform an intervention.

### SUMMARY

In accordance with a first aspect of the present disclosure, there is provided a system for tracking a surgical tool relative to a bone in computer-assisted surgery, comprising: a processing unit; and a non-transitory computer-readable memory communicatively coupled to the processing unit and comprising computer-readable program instructions executable by the processing unit for: tracking the surgical tool relative to the bone; merging virtual models of the surgical tool and the bone to the surgical tool and the bone in the tracking; calculating a location of a working end of the surgical tool relative to the bone using the tracking, in a concealed condition of the working end of the surgical tool relative to the bone; and outputting the location of the working end of the surgical tool relative to the bone.

Further in accordance with the first aspect, for example, the computer-readable program instructions are executable by the processing unit for indicating a proximity of the working end with a boundary of the bone.

Still further in accordance with the first aspect, for example, the computer-readable program instructions are executable by the processing unit for stopping the surgical tool when the working end is at the boundary of the bone.

Still further in accordance with the first aspect, for example, the computer-readable program instructions are executable by the processing unit for imaging and displaying the concealed working end of the surgical tool relative to the bone.

Still further in accordance with the first aspect, for example, the computer-readable program instructions are executable by the processing unit for imaging and displaying the concealed working end of the surgical tool relative to the bone in mixed reality on a face shield worn by the operator.

Still further in accordance with the first aspect, for example, the computer-readable program instructions are executable by the processing unit are for calibrating the surgical tool by obtaining video images of the surgical tool and its working end and processing same to size the model of the surgical tool.

Still further in accordance with the first aspect, for example, obtaining the video images of the surgical tool and its working end includes operating the surgical tool to image an amplitude of movement of the working end and size the amplitude of movement of the working end.

Still further in accordance with the first aspect, for example, the virtual model of the bone is obtained from pre-operative imaging.

Still further in accordance with the first aspect, for example, the virtual model of the surgical tool is obtained from a manufacturer file.

Still further in accordance with the first aspect, for example, the computer-readable program instructions are executable by the processing unit for controlling a robot arm as a function of a position and orientation of the tool.

Still further in accordance with the first aspect, for example, continuously outputting the location of the tool includes continuously outputting the location of the robot arm.

Still further in accordance with the first aspect, for example, tracking the surgical tool relative to the bone includes tracking the surgical tool relative to the bone from an image capture device at a point of view of an operator.

Still further in accordance with the first aspect, for example, tracking the surgical tool relative to the bone includes obtaining a second feed of tracking from a stationary image capture device.

Still further in accordance with the first aspect, for example, obtaining the video images from two different image capture devices includes prioritizing the video images from one of the image capture devices over the other of the image capture devices.

Still further in accordance with the first aspect, for example, tracking the surgical tool relative to the bone includes obtaining video images of the surgical tool relative to the bone, and wherein merging virtual models of the surgical tool and the bone to the surgical tool and the bone in the tracking includes processing the video images.

Still further in accordance with the first aspect, for example, calculating the location of the working end of the surgical tool relative to the bone includes using the video images processed.

Still further in accordance with the first aspect, for example, obtaining video images includes obtaining the video images in a lower frequency capture mode when the tool is distal to the bone, and in a higher frequency capture mode when the tool is proximal to the bone.

Still further in accordance with the first aspect, for example, a head-mounted device with at least one camera is provided for obtaining the video images.

Still further in accordance with the first aspect, for example, the head-mounted device has a display for outputting data associated with the position and orientation of the at least one object, the data being output in mixed reality.

In accordance with a second aspect of the present disclosure, there is provided a system for tracking a surgical tool relative to a bone in computer-assisted surgery, comprising: a processing unit; and a non-transitory computer-readable memory communicatively coupled to the processing unit and comprising computer-readable program instructions executable by the processing unit for: obtaining video images of the surgical tool relative to the bone, from a point of view on an operator; processing the video images to merge virtual models of the surgical tool and the bone to the surgical tool and the bone in the video images; calculating a location of a working end of the surgical tool relative to the bone using the video images processed, in a concealed condition of the working end of the surgical tool relative to the bone; and outputting the location of the working end of the surgical tool relative to the bone.

### DESCRIPTION OF THE DRAWINGS

Fig. 1 is a block diagram of a computer-assisted surgery (CAS) system with head-mounted navigation in accordance a variant with the present disclosure;
Fig. 2 is a perspective view and block diagram of a head-mounted device in accordance with another variant of the present disclosure;
Fig. 3A is a perspective view of a saw tool that may be used with the computer-assisted surgery (CAS) system with head-mounted navigation of Fig. 1;
Fig. 3B is a perspective view of a drilling tool that may be used with the computer-assisted surgery (CAS) system with head-mounted navigation of Fig. 1;
Fig. 4 is a perspective view of the CAS system of Fig. 1 during a resection of the tibia;
Fig. 5 is a perspective view of the CAS system of Fig. 1 during a resection of the tibia, with assistance from a robot arm; and
Fig. 6 is a perspective view of the CAS system of Fig. 1 during a resection of the tibia, with assistance from a patient-specific instrument such as a cut guide.

### DETAILED DESCRIPTION

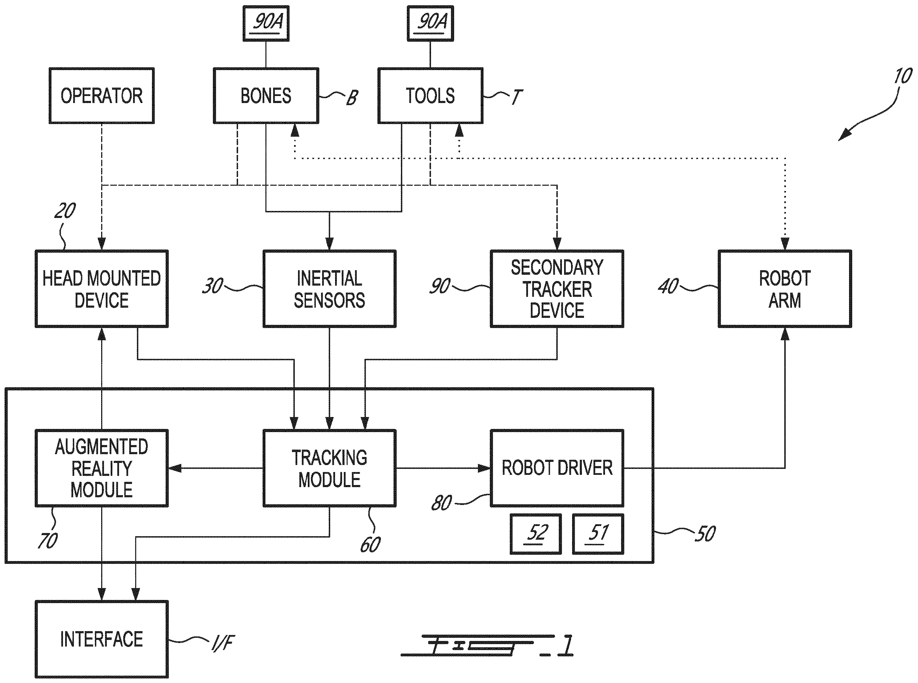

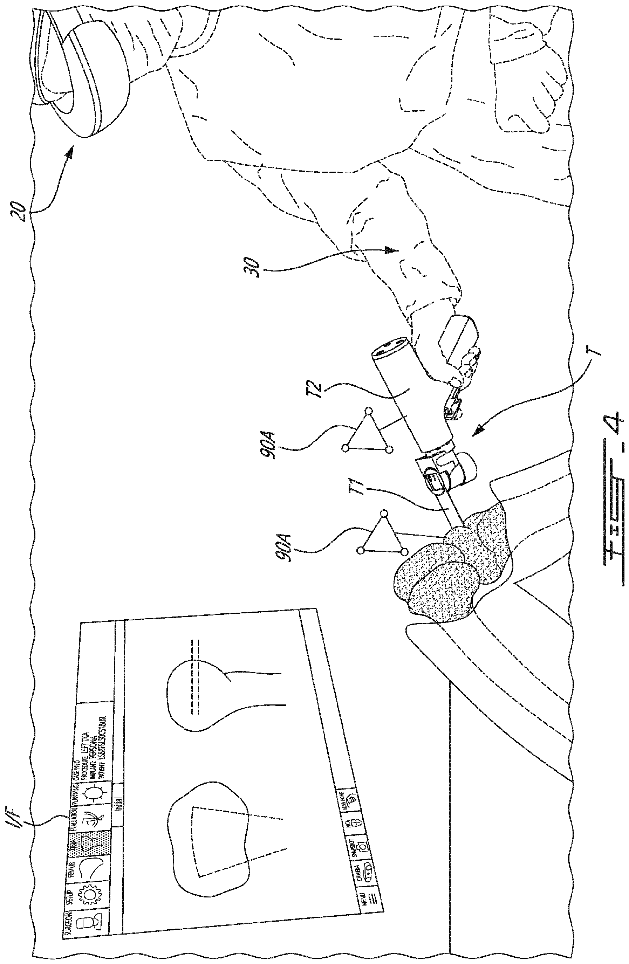

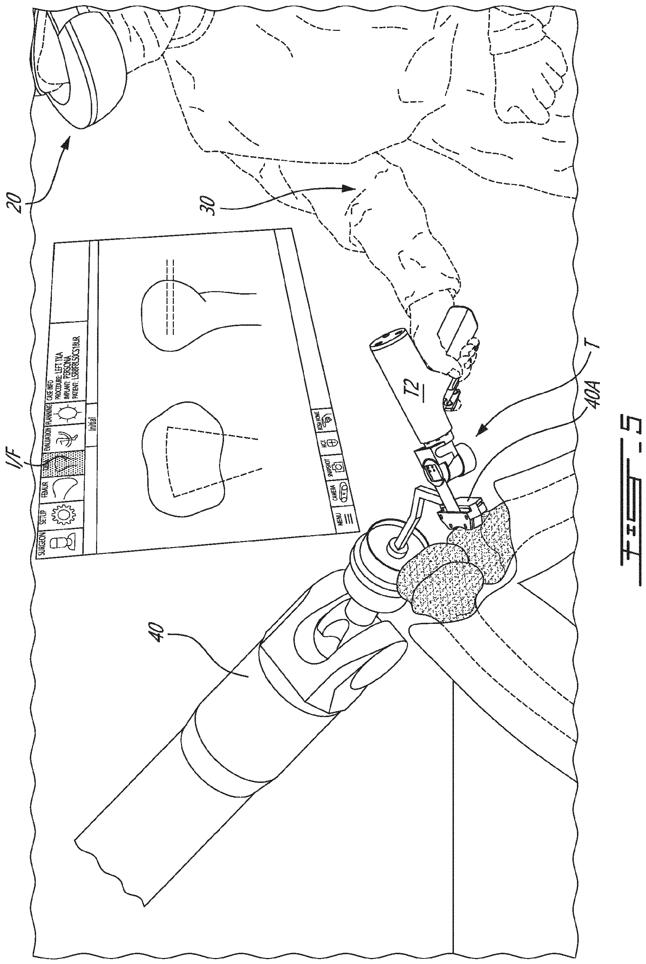

Referring to the drawings and more particularly to Fig. 1, computer-assisted surgery (CAS) system with head-mounted navigation with optional combined tracking is generally shown at 10, and is used to provide surgery assistance to an operator. For example, the CAS system 10 may be used to assist an operator wearing a head-mounted device with display in performing surgical maneuvers on a patient, such as surgical maneuvers associated with orthopedic surgery, including pre-operative analysis of range of motion, and implant assessment planning, as described hereinafter. In Figs. 4 and 5, the system 10 is shown relative to a patient's knee joint in supine decubitus, but only as an example. The system 10 could be used for other body parts, including non-exhaustively hip joint, spine, and shoulder bones, or in other types of surgery.

The CAS system 10 may be robotized in a variant, and has, may have or may be used with a head-mounted device or tracking device 20, one or more inertial sensor units 30, a robot arm 40, a CAS controller 50, a tracking module 60, an augmented reality module 70, a robot driver 80, and another tracking device 90, or any combination thereof:
- The head-mounted device 20 is worn by an operator, such as by the surgeon performing surgery, and may be referred to as head-mounted tracking device 20 as it has the capacity to capture images, such as in video format. The head-mounted device 20 may have a display screen to provide data to the wearer, though this may be optional in an embodiment. For simplicity, the expressions head-mounted tracking device 20 and head-mounted device 20 are used interchangeably in the present disclosure and figures. The head-mounted tracking device 20 may be used to provide a display in augmented/mixed and/or virtual reality to a user. The head-mounted tracking device 20 may also be tasked with taking images of the surgery, with the images being used for the tracking of patient tissue (such as bones) and tools, for instance as a video feed. The head-mounted tracking device 20 may also be used as an interface by which an operator may communicate commands to the CAS system 10.
- The inertial sensor units 30 may optionally be present, and positioned on the patient tissue and/or on the tool(s) and surgical instruments, to provide tracking data for the bones or tools. An inertial sensor unit 30 may also optionally be located on the head-mounted tracking device 20.
- The robot arm 40 may optionally be present as the working end of the system 10 - the CAS system 10 could also be non robotic -, and may be used to guide or to perform bone alterations as planned by an operator and/or the CAS controller 50 and as controlled by the CAS controller 50, or may be used to support a tool T that is operated by a surgeon. The robot arm 40 may also be configured for collaborative/cooperative mode in which the operator may manipulate the robot arm 40. For example, the tooling end, also known as end effector, and/or tool T at the tooling end may be manipulated by the operator while supported by the robot arm 40;
- The CAS controller 50 includes the processor(s) and appropriate hardware and software to run a computer-assisted surgery procedure in accordance with one or more workflows. The CAS controller 50 may include or operate the tracking module 60, the augmented reality module 70, and/or the robot driver 80 if present. Moreover, as described hereinafter, the CAS controller 50 may also drive the robot arm 40 through a planned surgical procedure, if the robot arm 40 is present;
- The tracking module 60 is tasked with determining the position and/or orientation of the various relevant objects during the surgery procedure, such as the bone(s) and tool(s), using data acquired by the head-mounted tracking device 20 (e.g., video feed), the inertial sensor unit(s) 30 (e.g., angular rates of change) if present, and optionally the tracking device 90 if present. The position and/or orientation may be used by the CAS controller 50 to control the robot arm 40;
- The augmented reality module 70 (a.k.a., mixed reality module) is provided to produce an augmented reality output to the operator, for instance for display in the head-mounted tracking device 20. The augmented reality module 70 may also produce other types of outputs, including a virtual reality output. The augmented reality module 70 may provide its output to displays other than head-mounted or wearable displays. For example, the augmented reality module 70 may produce an output for display on monitors of the CAS system 10, shown in Fig. 1 as interface I/F;
- The robot driver 80 is tasked with powering or controlling the various joints of the robot arm 40, if present, based on operator demands or on surgery planning.
- The tracking device 90 may optionally be used to track the patient tissue, instruments, and the robot arm 40. For example, the tracking device 90 may complement the tracking performed with the imaging done with the head-mounted tracking device 20, and may hence be referred to as a secondary tracking device. The tracking device 90 may employ camera technology similar to that of the head-mounted tracking device 20, such as depth cameras, with optional pattern projector, as described below, or may se a different imaging technology, to provide its video feed. The tracking device 90 may be said to be stationary. The tracking device 90 may be the primary tracking device 90, if the head-mounted tracking device 20 is absent or temporarily out of the line of the surgical scene, or with the head-mounted tracking device 20 providing complementary tracking capability. For example, the tracking device 90 may be a Navitracker^{®} device, that used with trackable references 90A, such as shown in Figs. 1 and 4. The tracking device 90 with trackable references 90A may be used in any of the embodiments described herein. The tracking device 90 may be the primary tracking system of the CAS system 10. In a variant, the tracking device 90 may be a Navitracker^{®} that tracks by triangulation trackable references 90A. In a variant, the trackable references 90A have retroreflective elements, such as spheres or tokens as shown, that passively reflect light. The retroreflective elements may be in a particular recognizable pattern, such as in a scalene triangle as shown, so as to be trackable in space for position and orientation. The trackable references 90A are typically fixed to an object such as a bone or tool (both in Figs. 1 and 4). In terms of invasiveness, the trackable reference 90A may be fixed to a bone with fasteners, such as screws or pins. As the trackable references 90A usually have a stem, the retroreflective elements are spaced apart from the bone, such that the trackable references 90A may contribute to limiting the invasiveness of the procedure.The tracking device 90 may use ultrasounds as well.

Other components, devices, systems, may be present, such as surgical instruments and tools T, the interfaces I/F such as displays, screens, computer station, servers, and the like etc.

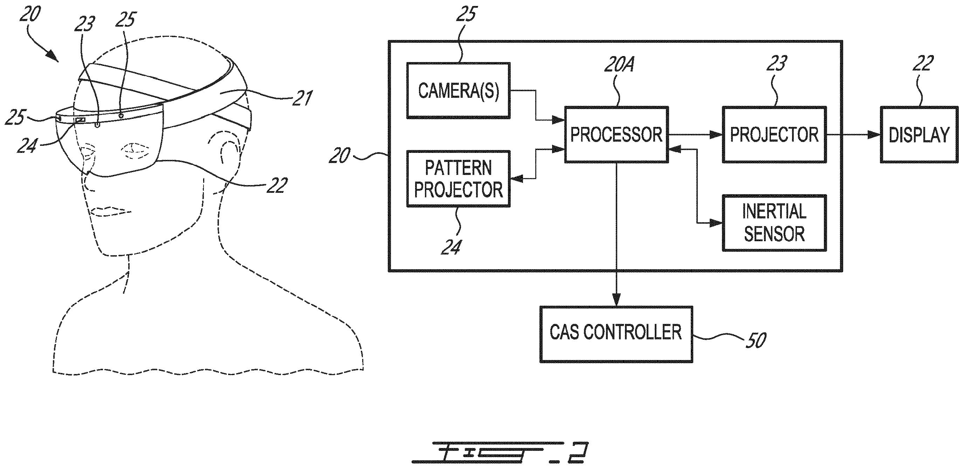

Referring to Fig. 2, a schematic example of the head-mounted tracking device 20 is provided. The head-mounted tracking device 20 may be as described in U.S. Patent No. 10,687,568, the contents of which are incorporated herein by reference, or may have other configurations. The head-mounted tracking device 20 described as a surgical helmet assembly in U.S. Patent No. 10,687,568 is well suited to be used in an augmented reality setting by its configuration. The head-mounted tracking device 20 may have a head enclosure 21 shaped to encircle a head of an operator. The head enclosure 21 may be straps, a rim, a helmet, etc. A face shield 22 may be mounted to a forehead or brow region of the head enclosure 21. The face shield 22 may be transparent to allow see-through vision by a user, but with the option of serving as a screen for augmented reality. Other components of the head-mounted tracking device 20 may include stabilizers, head band, a ventilation system with fan and vents, a light source, a rechargeable power source (e.g., a battery) etc.

The head-mounted tracking device 20 may consequently include a processor 20A and components to produce a mixed reality session. For instance, the head-mounted tracking device 20 may have an integrated projector 23 that may project data on the face shield 22, in a manner described below. Alternatively, the face shield 22 may be a screen having the ability to display images. As an example, the head-mounted tracking device 20 may be a HoloLens^{®}. In an embodiment, the face shield 22 is a display-like unit of the type that may be used in virtual reality, with camera(s) therein to create a mixed reality output using camera footage, such as an Oculus Rift^{®}, smartphone with head support, etc. The head-mounted tracking device 20 may include one or more orientation sensors, such as inertial sensor unit(s) (e.g., shown as 30), for an orientation of the head-mounted tracking device 20 to be known and tracked.

According to an embodiment, the head-mounted tracking device 20 is equipped to perform optical tracking of the patient tissue B, instruments T and/or robot arm 40, from a point of view (POV) of the operator. The head-mounted tracking device 20 may therefore have one or more imaging devices or apparatuses, to capture video images of a scene, i.e., moving visual images, a sequence of images over time. In a variant, the video images are light backscatter (a.k.a. backscattered radiation) used to track objects. In the present disclosure, the head-mounted tracking device 20 may be used to track tools and bones so as to provide navigation data in mixed reality to guide an operator based on surgery planning. Backscattered radiation can also be used for acquisition of 3D surface geometries of bones and tools.

The head-mounted tracking device 20 may produce structured light illumination for tracking objects with structured light 3D imaging. In structured light illumination, a portion of the objects is illuminated with one or multiple patterns from a pattern projector 24 or like light source. Structured light 3D imaging is based on the fact that a projection of a line of light from the pattern projector 24 onto a 3D shaped surface produces a line of illumination that appears distorted as viewed from perspectives other than that of the pattern projector 24. Accordingly, imaging such a distorted line of illumination allows a geometric reconstruction of the 3D shaped surface. Imaging of the distorted line of illumination is generally performed using one or more cameras 25 (including appropriate components such as e.g., lens(es), aperture, image sensor such as CCD, image processor) which are spaced apart from the pattern projector 24 so as to provide such different perspectives, e.g., triangulation perspective. In some embodiments, the pattern projector 24 is configured to project a structured light grid pattern including many lines at once as this allows the simultaneous acquisition of a multitude of samples on an increased area. In these embodiments, it may be convenient to use a pattern of parallel lines. However, other variants of structured light projection can be used in some other embodiments.

The structured light grid pattern can be projected onto the surface(s) to track using the pattern projector 24. In some embodiments, the structured light grid pattern can be produced by incoherent light projection, e.g., using a digital video projector, wherein the patterns are typically generated by propagating light through a digital light modulator. Examples of digital light projection technologies include transmissive liquid crystal, reflective liquid crystal on silicon (LCOS) and digital light processing (DLP) modulators. In these embodiments, the resolution of the structured light grid pattern can be limited by the size of the emitting pixels of the digital projector. Moreover, patterns generated by such digital display projectors may have small discontinuities due to the pixel boundaries in the projector. However, these discontinuities are generally sufficiently small that they are insignificant in the presence of a slight defocus. In some other embodiments, the structured light grid pattern can be produced by laser interference. For instance, in such embodiments, two or more laser beams can be interfered with one another to produce the structured light grid pattern wherein different pattern sizes can be obtained by changing the relative angle between the laser beams.

The pattern projector 24 may emit light that is inside or outside the visible region of the electromagnetic spectrum. For instance, in some embodiments, the emitted light can be in the ultraviolet region and/or the infrared region of the electromagnetic spectrum such as to be imperceptible to the eyes of the medical personnel. In these embodiments, however, the medical personnel may be required to wear protective glasses to protect their eyes from such invisible radiations, and the face shield 22 may have protective capacity as well. As alternatives to structured light, the head-mounted tracking device 20 may also operate with laser rangefinder technology or triangulation, as a few examples among others.

The head-mounted tracking device 20 may consequently include the cameras 25 to acquire backscatter images of the illuminated portion of objects. Hence, the cameras 25 capture the pattern projected onto the portions of the object. The cameras 25 are adapted to detect radiations in a region of the electromagnetic spectrum that corresponds to that of the patterns generated by the light projector 24. As described hereinafter, the known light pattern characteristics and known orientation of the pattern projector 24 relative to the cameras 25, are used by the tracking module 60 to generate a 3D geometry of the illuminated portions, using the backscatter images captured by the camera(s) 25. Although a single camera spaced form the pattern projector 24 can be used, using more than one camera 25 may increase the field of view and increase surface coverage, or precision via triangulation. The head-mounted tracking device 20 is shown as having a pair of cameras 25 is used.

The head-mounted tracking device 20 may also have one or more filters integrated into either or both of the cameras 25 to filter out predetermined regions or spectral bands of the electromagnetic spectrum. The filter can be removably or fixedly mounted in front of any given camera 25. For example, the filter can be slidably movable into and out of the optical path of the cameras 25, manually or in an automated fashion. In some other embodiments, multiple filters may be periodically positioned in front of a given camera in order to acquire spectrally resolved images with different spectral ranges at different moments in time, thereby providing time dependent spectral multiplexing. Such an embodiment may be achieved, for example, by positioning the multiple filters in a filter wheel that is controllably rotated to bring each filter in the filter wheel into the optical path of the given one of the camera 25 in a sequential manner.

In some embodiments, the filter can allow transmittance of only some predetermined spectral features of objects within the field of view, captured either simultaneously by the head-mounted tracking device 20 or separately by the secondary tracking device 90, so as to serve as additional features that can be extracted to improve accuracy and speed of registration.

More specifically, the filter can be used to provide a maximum contrast between different materials which can improve the imaging process and more specifically the soft tissue identification process. For example, in some embodiments, the filter can be used to filter out bands that are common to backscattered radiation from typical soft tissue items, the surgical structure of interest, and the surgical tool(s) such that backscattered radiation of high contrast between soft tissue items, surgical structure and surgical tools can be acquired. Additionally, or alternatively, where white light illumination is used, the filter can includes band pass filters configured to let pass only some spectral bands of interest. For instance, the filter can be configured to let pass spectral bands associated with backscattering or reflection caused by the bones, the soft tissue while filtering out spectral bands associated with specifically colored items such as tools, gloves and the like within the surgical field of view. Other methods for achieving spectrally selective detection, including employing spectrally narrow emitters, spectrally filtering a broadband emitter, and/or spectrally filtering a broadband imaging detector (e.g., the camera 25), can also be used. Another light source may also be provided on the head-mounted tracking device 20, for a secondary tracking option, as detailed below. It is considered to apply distinctive coatings on the parts to be tracked, such as the bone and the tool, to increase their contrast relative to the surrounding soft tissue.

In accordance with another embodiment, the head-mounted tracking device 20 may include a 3D camera(s), also shown as 25, to perform range imaging, and hence determine position data from the captured images during tracking - Fig. 2 showing two of such cameras 25 to enhance a depth perception. The expression 3D camera is used to describe the camera's capability of providing range data for the objects in the image or like footage it captures, but the 3D camera may or may not produce 3D renderings of the objects it captures. In contrast to structured light 3D imaging, range tracking does not seek specific illumination patterns in distance calculations, but relies instead on the images themselves and the 3D camera's capacity to determine the distance of points of objects in the images. Stated differently, the 3D camera for ranging performs non-structured light ranging, and the expression "ranging" is used herein to designate such non-structured light ranging. Such range tracking requires that the 3D camera be calibrated to achieve suitable precision and accuracy of tracking. In order to be calibrated, the head-mounted tracking device 20 may use a known visual pattern in a calibration performed *in situ,* at the start of the tracking, and optionally updated punctually or continuously throughout the tracking. The calibration is necessary to update the camera acquisition parameters due to possible lens distortion (e.g., radial, rotational distortion), and hence to rectify image distortion to ensure the range accuracy. Moreover, as described herein, tracking tokens with recognizable patterns (e.g., QR codes) may be used, with the patterns being used to determine a point of view (POV) of the cameras 25 of the head-mounted tracking device 20, via perspective deformation.

In a variant, the head-mounted tracking device 20 only has imaging capacity, for instance through cameras 25 (of any type described above), optionally pattern projector 24, without other components, such as face shield 22, etc.

Referring to Fig. 1, the optional inertial sensor units 30 are shown secured to the bones, but may also or alternatively be on instruments. The inertial sensor unit 30 may be known as a sourceless sensor, a micro-electromechanical sensor unit (MEMS unit), and has any appropriate set of inertial sensors (e.g., accelerometers, gyroscope) to produce tracking data in at least three degrees of rotation (i.e., the orientation about a set of three axes is tracked). The sensor unit 30 may be self-enclosed in a pod 31, that is connectable in an accurate and predetermined manner to a bone, an instrument T, the robot arm 40 if present. For example, brackets may also be used to immovably anchor the inertial sensor units 30 to the bones. The brackets may integrate additional features, such as a cut guide with varus-valgus adjustment capability, for example, in the form of dials, for example as described in U.S. Patent Application Publication No. 2019/0053813, incorporated herein by reference, the data of adjustment being navigated, i.e., tracked by the inertial sensor units 30. The inertial sensor units 30 may include a processor and a non-transitory computer-readable memory communicatively coupled to the processor and comprising computer-readable program instructions executable by the processor. Moreover, the inertial sensor units 30 may be self-contained, in that they may be pre-calibrated for operation, have their own powering or may be connected to a power source, and may have an interface, such as in the form of a display thereon (e.g., LED indicators).

Referring to Figs. 1 and 4, if present, the robot arm 40 may stand from a base, for instance in a fixed relation relative to the operating-room (OR) table supporting the patient, whether it is attached or detached from the table. The robot arm 40 has a plurality of joints and links, of any appropriate form, to support a tool T that interfaces with the patient. The end effector or tool head may indeed optionally incorporate a force/torque sensor for collaborative/cooperative control mode, in which an operator manipulates the tool T at the end of the robot arm 40. The robot arm 40 is shown being a serial mechanism, arranged for the tool head to be displaceable in a desired number of degrees of freedom (DOF). For example, the robot arm 40 controls 6-DOF movements of the tool head, i.e., X, Y, Z in the coordinate system, and pitch, roll and yaw. Fewer or additional DOFs may be present. For simplicity, only a generic illustration of the joints and links is provided, but more joints of different types may be present to move the tool head in the manner described above. The joints are powered for the robot arm 40 to move as controlled by the CAS controller 50 in the six DOFs, and in such a way that the position and orientation of the tool head in the coordinate system may be known, for instance by readings from encoders on the various joints. Therefore, the powering of the joints is such that the tool head of the robot arm 40 may execute precise movements, such as moving along a single direction in one translation DOF, or being restricted to moving along a plane, among possibilities. Such robot arms 40 are known, for instance as described in United States Patent Application Serial no. 11/610,728, and incorporated herein by reference. The head-mounted tracking device 20 and/or secondary tracking device 90 may be used for the tracking of the end effector of the robot arm 10, or other systems such as inertial sensor systems, e.g., an inertial sensor unit 30 may be on the robot arm 40.

Still referring to Fig. 1, the CAS controller 50 is shown in greater detail relative to the other components of the robotized CAS system 10. The CAS controller 50 has a processor unit 51 (one or more processors) and a non-transitory computer-readable memory 52 communicatively coupled to the processing unit 51 and configured for executing computer-readable program instructions executable by the processing unit 51 to perform some functions, such as tracking the patient tissue and tools, using the camera feed from the head-mounted tracking device 20 and/or from the tracking device 90, and the readings from the inertial sensor unit(s) 30. The CAS controller 50 may also control the movement of the robot arm 40. The CAS system 10 may comprise various types of interfaces I/F, for the information to be provided to the operator. In addition to the head-mounted tracking device 20, the interfaces I/F may include a monitor and/or screens including wireless portable devices (e.g., phones, tablets), audio guidance, LED displays, among many other possibilities. For example, the interface D includes a graphic-user interface (GUI) operated by the system 10. The CAS controller 50 may also display images captured by the cameras 25 of the head-mounted tracking device 20 and/or tracking device 90, for instance to be used in the collaborative/cooperative control mode of the system 10, or for visual supervision by the operator of the system 10, with augmented reality for example. The CAS controller 50 may drive the robot arm 40, if present, in performing the surgical procedure based on the surgery planning achieved pre-operatively. The CAS controller 50 may run various modules, in the form of algorithms, code, non-transient executable instructions, etc, in order to operate the CAS system 10 in the manner described herein. The CAS controller 50 may be part of any suitable processor unit(s), such as a personal computer or computers including laptops and desktops, tablets, server, cloud, etc.

The tracking module 60 may be a subpart of the CAS controller 50, or an independent module or system. The tracking module 60 receives from the head-mounted tracking device 20 and the tracking device 90 (if present) the video feed of the surgical scene, e.g., as backscatter images of the objects. The tracking module 60 may also concurrently receive tracking data (e.g., orientation data) from the inertial sensor unit(s) 30. In an embodiment, as the system 10 performs real-time tracking, the video images and the orientation data are synchronized, as they are obtained and processed simultaneously. Other processing may be performed to ensure that the video footage and the orientation data are synchronized.

The tracking module 60 processes the video images to track one or more objects, such as a bone, an instrument, etc. The tracking module 60 may determine the relative position of the objects, and segment the objects within the video images. In a variant, the tracking module 60 may process the video images to track a given portion of an object, that may be referred to as a landmark. The landmark may be different parts of the objects, objects on the objects, such as the pods 31, the tracking tokens 31A with recognizable patterns, etc.

The tracking module 60 may also be provided with models of the objects to be tracked. For example, the tracking module 60 may track bones and tools, and hence uses virtual bone models and tool models. The bone models may be acquired from pre-operative imaging (e.g., MRI, CT-scans), for example in 3D or in multiple 2D views, including with 2D X-ray to 3D bone model technologies. The virtual bone models may also include some image processing done preoperatively, for example to remove soft tissue or refine the surfaces that will be exposed and tracked. The virtual bone models may be of greater resolution at the parts of the bone that will be tracked during surgery, such as the knee articulation in knee surgery. The bone models may also carry additional orientation data, such as various axes (e.g., longitudinal axis, mechanical axis, etc). The bone models may therefore be patient specific. It is also considered to obtain bone models from a bone model library, with the data obtained from the video images used to match a generated 3D surface of the bone with a bone from the bone atlas. The virtual tool models may be provided by the tool manufacturer, or may also be generated in any appropriate way so as to be a virtual 3D representation of the tool(s). In a variant, the bone models do not include a full bone, but may only include a bone surface of a portion of a bone, such as for example the portion of the bone that is being resected. Such partial bone model, referred to herein as bone model, 3D bone model, virtual bone model, may include additional data, such as one or more axes. For example, in the case of a femur in knee surgery, the bone model may include a distal femur portion only. The bone model may also include a mechanical axis of the femur, that may be acquired pre-operatively via imaging, or intraoperatively through a calibration procedure (e.g., tracked movements of the femur relative to the pelvis). For a complete model, in a variant in which the model of the bone is partial, a bone atlas may be used to find an equivalent bone model, i.e., with corresponding features, which equivalent bone model may be merged with the partial bone model of the patient. All of the above variants of the bone model apply to any other bone described herein.

In a variant, the tracking module 60 may generate 3D models using the video images. For example, if the tracking module 60 can have video images of a tool, from 360 degrees, it may generate a 3D model that can be used for subsequent tracking. This intraoperative model may or may not be matched with pre-existing or pre-operative model of the tool.

Additional data may also be available, such as tool orientation (e.g., axis data and geometry). By having access to bone and tool models, the tracking module 60 may recognize an object in the image processing and/or may obtain additional information, such as the axes related to bones or tools. The image processing by the tracking module 60 may be assisted by the presence of the models, as the tracking module 60 may match objects from the video images with the virtual models.

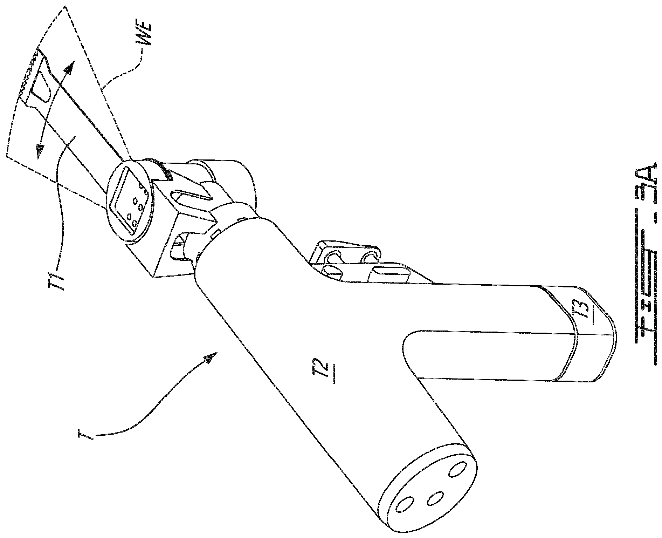

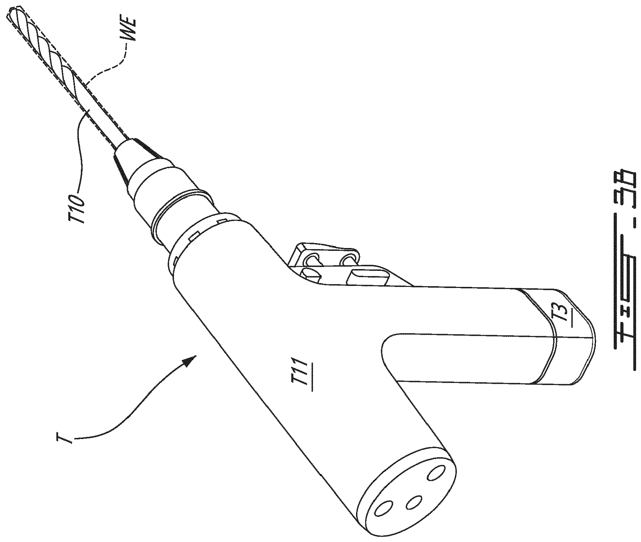

For example, two distinct tools T are shown in Figs. 3A and 3B. In Fig. 3A, the tool T is an oscillating saw having a blade T1 at a working end of the tool body T2. The tool body T2 is of the type that has a handgun shape, with actuating triggers. For example, the oscillating saw may be part of the X Series Power System^{™} ensemble, by Zimmer Biomet. The blade T1 has its cutting edge at its free end, and performs a back and forth reciprocating motion, such that it covers a working envelope WE. The arcuate path of the working envelope WE is that along which the blade T1 moves to resect the bone. In Fig. 3B, the tool T is a drill having a drill bit T10 at a working end of the tool body T11. The tool body T11 is also of the type that has a handgun shape, with actuating triggers. The drill bit T10 has a working envelope WE. Figs. 3A and 3B are representative of examples of tools that are used to perform alterations on bones, and multiple other tools could also be used and tracked with the CAS system 10. The working envelope WE of the tools T is relevant information, in that it is representative of the alterations on a bone. Accordingly, it may be tracked by the tracking module 60. In a variant, a calibration may be performed so as to model the working envelope WE. For example, the oscillating saw T of Fig. 3A may be actuated while being imaged, for the image processing to generate the working envelope WE from the amplitude of movement of the blade T1. The working envelope WE could also be accessible as a virtual model, for instance from the manufacturer. In any case, the working envelope WE may be relative to the body T2 (same principle applying to the drill T of Fig. 3B), with the body T2 having a fixed geometry and being viewable from the POV of the operator wearing the head mounted tracking device 20 during surgery.

The tools T of Figs. 3A and 3B may both have a power pack, shown as T3. The power pack T3 may include a battery for wireless operation of the tools T. Alternatively, the power pack T3 could be connected to an electric power source via a wire, or may be connected by tubing or piping to another motive source, such as hydraulic or pneumatic pressure network. The power pack T3 may include a controller unit, by which the tools T may be shut off, with the controller unit being communicatively coupled to the CAS controller 50 of the CAS system 10 (or to the processor 20A of the head-mounted tracking device 20), to receive commands therefrom, optionally. Thus, the power pack T3 (or any other part of the tool T) may have wireless communication capacity. Therefore, if one of the modules of the CAS system 10, such as the tracking module 60, identifies a stop condition, the CAS controller 50 may stop the operation of the tool. The stop condition may include the proximity of the working end WE of the tool T with soft tissue, a threshold zone around soft tissue that must not be impinged by the working end WE, the hidden boundary of a surface of the bone.

In a variant, the objects used as landmarks are parts of the bone and of the tool that are visible from the head-mounted tracking device 20. Stated differently, as the operator has a direct and proximal view of the surgical site, e.g., the bone being resected and the tool performing the resection, the footage from the POV of the head-mounted tracking device 20 is used by the tracking module 60 to navigate the tool T relative to the bone B. Despite the variation in POV of the camera(s) 25, the tracking module 60 uses the known dimensions of a landmark to track the objects in a referential system. The body of the tools T may for example be used as a basis for the tracking, as explained above, by way of the model of the body of the tool T (e.g., T2 in Fig. 3A, T11 in Fig. 3B). Even when parts of the tool T or bone B are concealed, or not in the line of sight (e.g., blade T1 in a cut), the use of the models of the tools T and bone B may enable to provide images of hidden parts of the tools T and bone B, such as on the GUIs of Figs. 4 and 5.

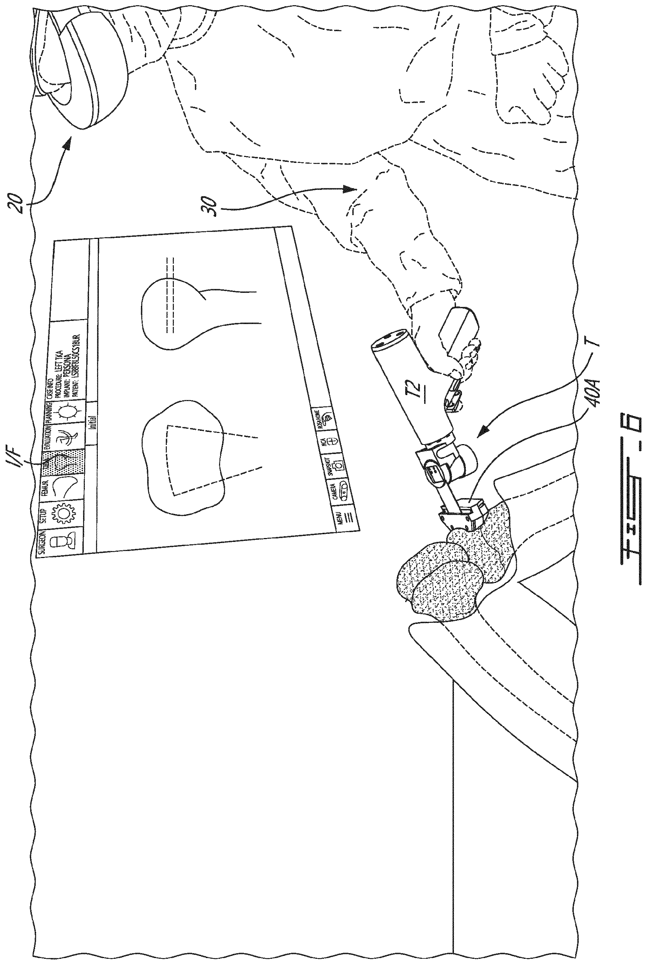

Optionally, it is considered to provide specific detectable landmarks on the tool(s) or bones to ensure the detectable landmarks will be properly imaged and detected by the tracking module 60. Indeed, in some instances when the view of some of the objects is limited, trackable references such as 90 in Fig. 4 may used, and this includes such trackable references in Fig. 5 and Fig. 6, and on the robot. As another example, tokens with given patterns, such as OR-code like labels, may be provided on the objects, such as on the bones or on the tools. In an embodiment, the tokens have an image that may be preprogrammed, or whose dimensions, are known or accessible by the tracking module 60. Accordingly, by seeing such tokens in the video images, the tracking module 60 may locate the objects in a spatial coordinate system. Again, the POV for the video images may move, with the objects serving as a referential for the tracking. The referential system may be that of the robot arm 20 (if present), with the camera tracking providing positional information for the referential system.

In matching the recognizable pattern and/or the 3D geometry to the bone models and tool models with the video images, the tracking module 60 may reduce its computation using different strategies. The bone model(s) B may have higher resolution for the parts of the bone that will be altered during surgery. The remainder of the bone may be limited to information on landmarks, such as axis orientation, center of rotation, midpoints, etc. A similar approach may be taken for the tool models C, with the focus and higher detail resolution being on parts of the tools that come into contact with the bone.

Moreover, considering that the camera(s) 25 may interrupt its line of sight with the object, the video feed from the tracker device 90 may complement that from the camera(s) 25 and/or supersede the video feed from the camera(s) 25. In another embodiment, the tracker device 90 is the primary tracking camera using any of the technologies described above for the head-mounted tracking device 20. Therefore, the tracking module 60 continuously updates the position and/or orientation of the patient bones and tools in the coordinate system using the video feed from the camera(s) 25 and/or tracking device 90 to track objects in position and orientation.

In an embodiment with structured light projection, the tracking module 60 receives the backscatter images from the camera(s) 25 or from the tracking device 90, as a result of the structured light projection from the projector 24. In another embodiment, the tracking module 60 receives the video images from the camera 25 in a depth camera configuration, and may ensure take steps to calibrate the camera(s) 25 or tracking device 90 for ranging to be done from the acquired images. An initial calibration may be done using a calibration pattern, such as that in tokens. The calibration pattern is placed in the light of sight of the camera(s) 25 or tracking device 90 such that it is imaged. The calibration pattern is any appropriate shape and configuration, but may be a planar recognizable pattern with high contrast, or given landmarks of a bone, or geometry of tool. Other items can be used for the calibration, including the body of a tool T, whose geometry may be programmed into or may be accessed by the tracking module 60. The tracking module 60 stores a virtual version of the calibration pattern, including precise geometrical data of the calibration pattern. The tracking module 60 therefore performs a correspondence between imaged and virtual calibration patterns. The correspondence may entail calculating the mapping function between landmarks on the planar imaged calibration pattern and the virtual calibration pattern. This may include a projection of the calibration patterns on one another to determine the distortion characteristics of the images of the camera(s) 25 or tracking device 90, until the rectification values are determined by the tracking module 60 to correct the images of camera. This calibration may be repeated punctually through the procedure, for instance based on the camera updating requirements. It may require that the camera be used in conjunction with a calibration reflective surface whose position and orientation relative to the camera is known. The calibration may be automatically performed by the CAS system 10.

The tracking module 60 may therefore perform a 3D geometry image processing, using the known patterns of structured light, or calibrated camera images, video feed, etc, along with the known shape of the virtual bone model(s) and/or tool model(s), optionally with QR tokens or trackable references 90A, and generate 3D images from the tracking, using for examples the pre-operative models. Moreover, a generated 3D geometry may be located in the X, Y, Z, coordinate system using the tracking of landmarks on the bones or tools to set the coordinate system on the bones. Therefore, the tracking module 60 may generates an image or 3D geometry of the landmarks on the object(s) being illuminated. Then, using the virtual models and/or of the bone(s) and tool(s), respectively, the tracking module 60 can match the image or 3D geometry with the virtual models of the landmarks. Consequently, the tracking module 60 determines a spatial relationship between the landmarks being imaged and the preoperative 3D models, to provide a dynamic (e.g. real time or quasi real time) intraoperative tracking of the bones relative to the tools, in spite of tool portions and bone surfaces not being visible from the POV of the operator.

In an embodiment, the position and orientation of the surgical tool calculated by the tracking module 60 may be redundant over the tracking data provided by the robot driver 80 and robot arm sensors, if the robot arm 40 is used. However, the redundancy may assist in ensuring the accuracy of the tracking of the surgical tool. For example, the redundancy is used as a safeguard against disruption of the line of sight between the head-mounted tracking device 20 and the surgical site, for instance if the operator looks away. The redundancy may also allow the reduction of frequency of image processing for the surgical tool. Also, the tracking of the tool using the tracking module 60 may be used to detect any discrepancy between a calculated position and orientation of the surgical tool T through the sensors on the robot arm 40, and the actual position and orientation of the surgical tool. For example, an improper mount of the tool T into the chuck of the robot arm 40 could be detected from the output of the tracking module 60, when verified with the position and orientation from the robot driver 80 (e.g., obtained from the encoders on the robot arm 40). The operator may be prompted to verify the mount, via the interface I/F or head-mounted tracking device 20.

The camera 25 and/or tracking device 90 may continuously capture images, for the tracking module 60 to perform a continuous tracking of the objects. The terms video feed, image feed, video, may be used to describe the capture of images by the head-mounted tracking device 20, and optionally by the tracking device 90, and entails a capture of frames over a time period, in contrast to a single image capture. The frequency of capture may vary according to different factors. For example, there may be different phases during the surgical workflow, some in which the tracking requires a more dynamic update (i.e., higher frequency), and some in which tracking updates are less important. Another factor that may affect the image capture frequency is the fixed relation of the objects. For example, once the tracking module 60 identifies a landmark and tracks a bone from the images, the frequency capture by the camera 25 and/or tracking device 90 may be reduced if the bone is fixed or if no maneuvers are performed, if the bone alterations have not yet begun. Also, when both a tool and a bone are tracked, the frequency capture may be reduced when the tool and the bone are spaced from one another by a given distance, and increased as the proximity between the tool and the bone is increased. The tracking module 60 may drive the camera 25 and/or tracking device 90 in order to control the frequency. For example, the tracking module 60 may adapt the frequency using the surgical planning, e.g., anticipating upcoming steps in the workflow, etc. The tracking module 60 may consequently toggle between a lower frequency capture mode and a higher frequency capture mode, for example. The lower frequency capture mode may be in instances in which the tool is at a given distance from the bone, and is not driven to alter the bone. The lower frequency capture mode may also be operated when the objects are in a fixed relation relative to one another. Other modes are contemplated. The tracking module 60 may output the data directly on the interfaces I/F.

The augmented reality module 70 may be present in the CAS controller 50 and may produce an augmented reality (AR) output to the operator, for instance for display in the head-mounted tracking device 20. The augmented reality module 70 may also produce other types of outputs, including a virtual reality output. The augmented reality module 70 may provide its output to displays other than head-mounted tracking device 20. For example, the augmented reality module 70 may produce an output for display on monitors of the CAS system 10.

As seen in Figs. 4-6, the floating GUIs shown as interface I/F may be projected or displayed onto the face shield 22 of the head-mounted tracking device 20, so as to be in the line of sight of the wearer, and/or may be duplicated or alternatively output on the interfaces I/F. In an embodiment, the head-mounted tracking device 20 projects the AR images in parts of the face shield 22 so as not to interfere with the line of sight between the wearer and bone or instrument, or to augment the objects being viewed, such as by adding axes to the bones, simulating the position of implants, or of the concealed tooling end of the tool T, among other possibilities.

In a variant, the wearer may also interface with the CAS controller 50 using the AR output from the head-mounted tracking device 20. For example, when looking away from the surgical site, or at given instances during the surgical workflow, the head-mounted tracking device 20 may display virtual touch zones, and may track with its camera 25 the wearer's arm reaching any such touch zone. The head-mounted tracking device 20 and CAS controller 50 may trigger an action based on the touch zone activation.

Accordingly, while the data provided by the augmented reality module 70 could be displayed on separate monitors or like interfaces, the display of the images in augmented reality may minimize the movements to be made by the wearer, increase the rapidity of access to information and/or provide information that may not otherwise be available.

Still referring to Fig. 1, the CAS controller 50 may have the robot driver module 80, if a robot arm 40 is present in the CAS system 10. The robot driver module 80 is tasked with powering or controlling the various joints of the robot arm 40. There may be some force feedback provided by the robot arm 40 to avoid damaging the bones. The robot driver module 80 may perform actions based on a surgery planning. The surgery planning may be a module programmed specifically for any given patient, according to the parameters of surgery desired by an operator such as an engineer and/or surgeon. The parameters may include geometry of selected, planned bone cuts, planned cut depths, sequence or workflow of alterations with a sequence of surgical steps and tools, tools used, etc.

Referring now to Figs. 4 and 5, a contemplated use of the CAS system 10 is shown, as occurring during a bone model procedure in surgery, between femur and tibia, for ease of visual representation.

In the variant of Fig. 4, a surgeon or other operator has the head-mounted tracking device 20 with tracking technology as described above, with camera(s) 25 to perform point of view tracking. Optionally, though not shown, the tracking device 90 may be used as well, with the trackable references 90A as shown. Alternatively, tracking patterns may be on the bones and/or tools, though they are optional, or could be elsewhere. The tracking using the video feed from the head-mounted tracking device 20 allows the CAS system 10 to limit obstructions as the operator usually maintains a direct unobstructed POV with the surgical site.

In the variant of Fig. 5, a surgeon or other operator has the head-mounted tracking device 20 with tracking technology as described above, with camera(s) 25 to perform point of view tracking, and with the robot arm 40 having a cut guide 40A to assist in locating the cut plane for the surgeon or other operator manipulating the tool T. Again, though not shown, the tracking device 90 may be used as well, such as with trackable references 90A. Tracking patterns may be on the bones and/or tools, though they are optional, or could be elsewhere. The tracking using the video feed from the head-mounted tracking device 20 allows the CAS system 10 to limit obstructions as the operator usually maintains a direct unobstructed POV with the surgical site.

In the variant of Fig. 6, a surgeon or other operator has the head-mounted tracking device 20 with tracking technology as described above, with camera(s) 25 to perform point of view tracking, and with cut guide 40A being a patient-specific cut guide, also known as PSI cut guide, in that it has contour matching surface(s) to abut a bone in complementary unique engagement, i.e., the mating can only be done at one location. The cut guide 40A is provided to assist in locating the cut plane for the surgeon or other operator manipulating the tool T, and thus to ensure that the tool T follows a desired trajectory, such as along a plane for the blade T1. The PSI cut guide 40A (or any other PSI, i.e., patient specific instrument) may be associated with a virtual 3D model to fabricate it, as a function of a virtual 3D model of the bone. More particularly, in the conception of the PSI, its virtual model may be applied to the virtual model of the bone, emulating with precision the subsequent PSI 40A and bone relation. Accordingly, in the variant of Fig. 6, the PSI cut guide 40A may be navigated by the point of view tracking, using its 3D model and that of the bone, as assembled. The CAS system 10 may have access to the virtual 3D model of the assembly of the PSI cut guide 40A on the bone in the complementary unique engagement, and thus may use this to calculate the penetration of the tool and working end WE, using for example the visual tracking of the PSI 40A due to its more complete and distinct geometry than that of the bone. Again, though not shown, the tracking device 90 may be used as well., such as with trackable references 90A Tracking patterns may be on the bones and/or tools, though they are optional, or could be elsewhere. The tracking using the video feed from the head-mounted tracking device 20 allows the CAS system 10 to limit obstructions as the operator usually maintains a direct unobstructed POV with the surgical site.

The CAS system 10 of Figs. 4, 5 and/or 6 may be used in the following manner. During surgery, with the head-mounted tracking device 20, the CAS system 10 may obtain video images of the surgical tool T relative to the bone, from a point of view on an operator, such as the surgeon, or from a stationary tracking system, such as the tracking device 90. The video images may include the imaging of the trackable references 90A, or any other trackable component, or just images of the objects. The video images are processed by the CAS system 10, to merge virtual models of the surgical tool and the bone to the surgical tool and the bone (including trackable references 90A) in the video feed. The virtual models of the surgical tool and of the bone (and optionally of the bone and PSI in the example of Fig. 6) may be pre-operative models resulting from different imaging modalities, may be manufacturer CAD models for tools, may be the result of a calibration, oy any combination thereof, and may be limited to portions of a bone as described above, etc. The CAS system 10 may calculate and continuously output a location of a working end of the surgical tool relative to the bone, in a concealed condition of the working end of the surgical tool relative to the bone, as a bone altering operation occurs, such as plane resection, bone drilling, etc. As shown in Figs. 4, 5 and 6, the CAS system 10 may therefore display the working end of the surgical tool relative to the bone, even though the working end is concealed or hidden, notably in the circumstances of a limited incision in minimally invasive surgery. This includes a display of the working envelope WE of the surgical tool, as shown in Fig. 4. Therefore, the operator may have live virtual footage of the working end of the surgical tool relative to the bone penetrating the bone on an interface I/F. The CAS system 10 may hence detect stop conditions, to alarm the operator, and/or to stop the tool T, for instance by indicating a proximity of the working end with a boundary of the bone. In parallel, the CAS system 10 may image or display the concealed working end of the surgical tool relative to the bone, such as on the head-mounted tracking device 20, e.g., in mixed reality on a face shield worn by the operator, or on other interfaces. The CAS system 10 may calibrate the tool(s) before the bone altering maneuvers, such as by obtaining video images of the surgical tool and its working end and processing same to size the model of the surgical tool. The tool may be operated to image an amplitude of movement of the working end and size the amplitude of movement of the working end. In the variant of Fig. 5, the robot arm 40 may be controlled as a function of a position and orientation of the tool and/or through the video images of a tool such as a cut block being processed. Thus, the CAS system 10 may continuously output the location of the robot arm 40 (or of a tool supported by the robot arm 40, such as the illustrated cutting block). The CAS system may obtain a second feed of video images from a stationary image capture device, such as the tracker device 90. The CAS system 10 may prioritize the video images from one of the image capture devices over the other of the image capture devices, and/or may obtain the video images in a lower frequency capture mode when the tool is distal to the bone, and in a higher frequency capture mode when the tool is proximal to the bone.

A calibration of the system 10 vis à vis the tibia of the patient is achieved. The calibration may include one or more of steps and/or substeps, such as obtaining images of the tibia, obtaining dynamic motion data, digitizing or registration bone landmarks, a bone model (e.g., 3D virtual, 2D images) by the head-mounted tracking device 20, creating a referential system by which tibial axes are recorded for subsequent tracking by the head-mounted device 20. Thus, once calibration is achieved, a referential system incorporating landmarks of the tibia exists, such that the video feed of the tibia from the head-mounted tracking device 20 allows the CAS controller 50 to track the tibia in space for position and/or orientation.

When the tibial cut is made/the tibial cut is tracked, it may be performed in different ways, depending from different points of view. If the system 10 includes the robot arm 40, the system 10 may perform the cutting action. The location of the cut may include determining a position and/or orientation of the cut to be made based on a selected implant geometry (and thickness along the cranial-caudal axis). The determination may be defined as obtaining such data from the surgeon and/or making recommendations to the surgeon. With the position and/or orientation known, the cut may be virtually placed in the referential system of the tibia, such that tracking using the head-mounted device 20 may subsequently be performed. Accordingly, tracking guidance may be output for the cut to be made, including an image of the concealed working end of the tool relative to the periphery of the bone. Tracking guidance may be to the robot arm 40 or to the surgeon or staff member. In terms of tracking guidance to the surgeon, the head-mounted device 20 may be used to track a cutting tool as shown in Figs. 4, 5 and 6. For example, the CAS controller 50 may be provided with the geometry of the tool (and cut guide such as in Fig. 6), and the tool may be tracked relative to the referential system using the head-mounted device 20 and imaging capacity thereof. Additional information may be available, including varus-valgus as a function of the cut. In a variant, the cut is made using the cut guide 40A of Fig. 5 as supported by the robot arm 40, with varus-valgus adjustment capability, or the PSI cut guide 40A as in Fig. 6.

Accordingly, by the image feed provided by the head-mounted tracking device 20, though optional, the CAS system 10 may have a close up view on the surgical site. With the image processing capacity of the CAS system 10, the tracking module 60 may precisely calculate the spatial positioning of the concealed working end of the tool T relative to hidden surfaces of the bone, and provide a virtual live representation thereof. The tracking module 60 may thus be configured to detect stop conditions, such as the proximity of soft tissue that could be damaged by the working end WE. The tracking module 60 may send alarm signals to the operator, image the proximity on the GUIs or like interfaces I/F, and shut off the tools T, to prevent substantial soft tissue damage. The tracking module 60 may also operate a deceleration profile for the working end WE to decelerate its movements, within a threshold zone in proximity to the contour of the bone.

The present disclosure refers to the system 10 as performing continuous tracking. This means that the tracking may be performed continuously during discrete time periods of a surgical procedure. Continuous tracking may entail pauses, for example when the bone is not being altered. However, when tracking is required, the system 10 may provide a continuous tracking output, with any disruption in the tracking output triggering an alarm or message to an operator. The frequency of tracking may vary.

The CAS system 10 may generally be described as a system for tracking one or more objects in computer-assisted surgery using a processing unit and a non-transitory computer-readable memory communicatively coupled to the processing unit and comprising computer-readable program instructions executable by the processing unit. The computer-readable program instructions executable may be for obtaining video images of the surgical tool relative to the bone, from a point of view on an operator; processing the video images to merge virtual models of the surgical tool and the bone to the surgical tool and the bone in the video images; calculating a location of a working end of the surgical tool relative to the bone using the video images processed, in a concealed condition of the working end of the surgical tool relative to the bone; and outputting the location of the working end of the surgical tool relative to the bone.

A method for tracking at least one object with a computer-assisted surgery system may include computer-readable program instructions executable by the processing unit may be for: obtaining video images of the surgical tool relative to the bone, from a point of view on an operator; processing the video images to merge virtual models of the surgical tool and the bone to the surgical tool and the bone in the video images; calculating a location of a working end of the surgical tool relative to the bone using the video images processed, in a concealed condition of the working end of the surgical tool relative to the bone; and outputting the location of the working end of the surgical tool relative to the bone.

## Claims

1. A system for tracking a surgical tool relative to a bone in computer-assisted surgery, comprising:
a processing unit; and
a non-transitory computer-readable memory communicatively coupled to the processing unit and comprising computer-readable program instructions executable by the processing unit for:
tracking the surgical tool relative to the bone;
merging virtual models of the surgical tool and the bone to the surgical tool and the bone in the tracking;
calculating a location of a working end of the surgical tool relative to the bone using the tracking, in a concealed condition of the working end of the surgical tool relative to the bone; and
outputting the location of the working end of the surgical tool relative to the bone.

2. The system according to claim 1, wherein the computer-readable program instructions are executable by the processing unit for indicating a proximity of the working end with a boundary of the bone.

3. The system according to any one of claims 1 to 2, wherein the computer-readable program instructions are executable by the processing unit for stopping the surgical tool when the working end is at the boundary of the bone.

4. The system according to any one of claims 1 to 3, wherein the computer-readable program instructions are executable by the processing unit for imaging and displaying the concealed working end of the surgical tool relative to the bone.

5. The system according to claim 4, wherein the computer-readable program instructions are executable by the processing unit for imaging and displaying the concealed working end of the surgical tool relative to the bone in mixed reality on a face shield worn by the operator.

6. The system according to any one of claims 1 to 5, wherein the computer-readable program instructions are executable by the processing unit are for calibrating the surgical tool by obtaining video images of the surgical tool and its working end and processing same to size the model of the surgical tool.

7. The system according to claim 6, wherein obtaining the video images of the surgical tool and its working end includes operating the surgical tool to image an amplitude of movement of the working end and size the amplitude of movement of the working end.

8. The system according to any one of claims 1 to 7, including obtaining the virtual model of the bone from pre-operative imaging.

9. The system according to any one of claims 1 to 8, including obtaining the virtual model of the surgical tool from a manufacturer file.

10. The system according to any one of claims 1 to 9, wherein the computer-readable program instructions are executable by the processing unit for controlling a robot arm as a function of a position and orientation of the tool and wherein, optionally, continuously outputting the location of the tool includes continuously outputting the location of the robot arm.

11. The system according to any one of claims 1 to 10, wherein tracking the surgical tool relative to the bone includes tracking the surgical tool relative to the bone from an image capture device at a point of view of an operator.

12. The system according to claim 11, wherein tracking the surgical tool relative to the bone includes obtaining a second feed of tracking from a stationary image capture device.

13. The system according to any one of claims 1 to 12, wherein tracking the surgical tool relative to the bone includes obtaining video images of the surgical tool relative to the bone, and wherein merging virtual models of the surgical tool and the bone to the surgical tool and the bone in the tracking includes processing the video images.

14. The system according to claim 13, wherein calculating the location of the working end of the surgical tool relative to the bone includes using the video images processed.

15. A system for tracking a surgical tool relative to a bone in computer-assisted surgery, comprising:
a processing unit; and
a non-transitory computer-readable memory communicatively coupled to the processing unit and comprising computer-readable program instructions executable by the processing unit for:
obtaining video images of the surgical tool relative to the bone, from a point of view on an operator;
processing the video images to merge virtual models of the surgical tool and the bone to the surgical tool and the bone in the video images;
calculating a location of a working end of the surgical tool relative to the bone using the video images processed, in a concealed condition of the working end of the surgical tool relative to the bone; and
outputting the location of the working end of the surgical tool relative to the bone.
